# EUROPÄISCHE PATENTSCHRIFT

(11) **EP 3 096 818 B1**
(45) Veröffentlichungstag und Bekanntmachung des Hinweises auf die Patenterteilung: **16.09.2020**
(21) Anmeldenummer: 15700862.4
(22) Anmeldetag: 19.01.2015
(51) Int. Cl.: A61M 5/168

(54) **VERFAHREN UND VORRICHTUNG ZUR KONTROLLE DES DURCHFLUSSES DURCH EINE MEDIZINISCHE INFUSIONSLEITUNG**
METHOD AND APPARATUS FOR FLOW CONTROL THROUGH A MEDICAL INFUSION LINE
SYSTÈME ET METHOD POUR CONTRÔLER LE DÉBIT DANS DES CONDUITS D'INFUSION MÉDICAL

(30) Priorität: 23.01.2014 DE 102014201258
(43) Veröffentlichungstag der Anmeldung: 30.11.2016
(73) Patentinhaber: B. Braun Melsungen AG, 34212 Melsungen (DE)
(72) Erfinder: HASLBECK, Karsten, 34212 Melsungen (DE)
(74) Vertreter: Patentanwälte Ruff, Wilhelm, Beier, Dauster & Partner mbB
(86) Internationale Anmeldenummer: PCT/EP2015/050899
(87) Internationale Veröffentlichungsnummer: WO 2015/110387

(56) Entgegenhaltungen:
- WO-A1-2011/020201
- US-A- 2 350 712
- US-A- 3 120 125
- US-A- 4 559 454
- US-A1- 2011 152 762
- US-A1- 2012 150 113

## Beschreibung

Die Erfindung betrifft eine Vorrichtung zur Kontrolle des Durchflusses durch eine medizinische Infusionsleitung, an deren einem Ende eine Fluidförderpumpe vorgesehen ist und deren anderes Ende einem Patienten zugeordnet ist, wobei die Infusionsleitung einen Hauptflussweg von der Fluidförderpumpe zu dem Patientenende bildet.

Derartige Infusionsleitungen werden dazu verwendet, einem Patienten ein Medikament in fluider Form kontinuierlich zuzuführen. Beispielsweise werden Medikamente kontinuierlich in geringer Dosierung zugeführt. Dabei wird das Fluid mit äußerst geringen Flussraten im Bereich zwischen 0,5 und 500 ml pro Stunde gefördert. Falls die Pumpe ausfällt oder aus einem anderem Grund das Fluid nicht gefördert wird, kann dies erst nach fortgeschrittener Infusionszeit festgestellt werden. Insbesondere bei niedrigen Flussraten war ein Gewichts- oder Volumenabnahme des Flüssigkeitsreservoirs der Pumpe kaum sichtbar und mit praktikablem Aufwand nicht messbar. Es besteht daher ein Bedarf, auf schnelle und einfache Weise feststellen zu können, ob Fluid durch die Infusionsleitung gefördert wird. Bisher bekannte Infusionsleitungvorrichtungen und Durchflusskontrollen sind beispielsweise in US 2012/150113, WO 2011/020201 und US 2011/152762 offenbart.

Bisher bekannte Messverfahren basieren auf dem Grundgedanken, die Durchflussrate in der Strömung zu messen. Bei den geringen Flussraten in der Infusionstherapie oder bei der Schmerzmittelinfusion scheiden mechanische Lösungen aus, bei denen die Flussrate durch von der Strömung mechanisch bewegte Teile bestimmt wird. Die Flussraten reichen nicht aus, um mechanische Teile zu bewegen. Vielmehr werden diese von dem Fluid umströmt, ohne dass ein Impulsübertrag eine mechanische Bewegung ermöglicht. Alternativ finden elektronische Lösungen Anwendung, bei denen der Durchfluss in der Strömung mit Hilfe elektronischer Sensoren ermittelt wird. Diese elektronischen Lösungen sind zum einen teuer und zum anderen wird eine externe Energiequelle benötigt. Eine elastomere Infusionspumpe sollte jedoch unabhängig von externen Energiequellen arbeiten können.

Der Erfindung liegt die Aufgabe zugrunde, eine technisch einfache und zuverlässige Kontrolle des Durchflusses in einer medizinischen Infusionsleitung zu ermöglichen.

Die erfindungsgemäße Vorrichtung wird definiert durch den Patentanspruch 1.

Durch die Infusionsleitung führt der Hauptflussweg von der Fluidförderpumpe zu dem Patientenende. Das Patientenende ist mit einem Konnektor versehen, der zum Anschluss des Patienten oder in den Patienten eingeführten Komponenten dient. In technischer Hinsicht handelt es sich bei dem Patientenende also um einen Anschluss für den Patienten. Während es nach den bislang bekannten Durchflusskontrollverfahren erforderlich war, den Durchfluss innerhalb der Strömung in dem Hauptflussweg zu erkennen, basiert die Erfindung auf dem Gedanken, die Fluidströmung in ein separates Messreservoir umzuleiten, wobei der Hauptflussweg in Richtung des Patientenendes vor oder nach der Abzweigung des Messflussweges eine Flussdrossel aufweist. Die Fluidförderpumpe wird dabei unverändert weiter betrieben und die gesamte Energie der Fluidströmung kann zur Kontrolle des Durchflusses genutzt werden. Dabei kann zunächst das beim Betrieb der Pumpe aus dem Hauptflussweg in das Messreservoir einströmende Fluid als Hinweis auf die grundsätzliche Funktionsfähigkeit der Pumpe angesehen werden.

Zur Kontrolle des Durchflusses wird erfindungsgemäß der Hauptflussweg unterbrochen, wobei zwei Varianten denkbar sind:
Zum einen kann der Hauptflussweg zwischen der Fluidförderpumpe und dem Abzweig des Messflussweges von dem Hauptflussweg unterbrochen werden. Dabei wird also der Hauptflussweg in Flussrichtung vor dem Messreservoir unterbrochen und die Fluidförderpumpe fördert kein weiteres Fluid in das Messreservoir und zu dem Patientenende hin. Nach derartigem Unterbrechen des Hauptflussweges wird das Fluid in dem Messreservoir beobachtet. Wenn Fluid ausströmt ist die Infusionsleitung in Richtung des Patienten durchgängig und der Durchfluss zum Patienten gewährleist. Wenn kein Fluid aus dem Messreservoir ausströmt, ist dies ein Hinweis darauf, dass die Infusionsleitung in Richtung des Patientenendes und/oder der Durchfluss zu dem Patienten unterbrochen oder beeinträchtigt ist. Bei dieser ersten Variante ist eine Flussdrossel in der Infusionsleitung entlang des Hauptflussweges zwischen der Abzweigung des Messflussweges und dem Patientenende vorgesehen. Diese Variante ist aus dem Stand der Technik bekannt und nicht Teil der Erfindung.

Bei der erfindungsgemäßen Variante wird der Hauptflussweg zwischen der Abzweigung des Messflussweges und dem Patientenende unterbrochen, das heißt also in Flussrichtung hinter der Abzweigung des Messflussweges und dem Messreservoir. Hierbei ist eine Flussdrossel in der Infusionsleitung entlang des Hauptflussweges zwischen der Fluidförderpumpe und der Abzweigung des Messflussweges angeordnet. Die Flussdrossel ist dabei also in Flussrichtung vor der Abzweigung des Messflussweges und des Messreservoirs vorgesehen. Bei Unterbrechen des Hauptflussweges in Flussrichtung hinter der Abzweigung des Messflussweges wird das von der Fluidförderpumpe durch die Infusionsleitung geförderte Fluid vollständig in den Messflussweg und in das Messreservoir einströmen. Die Menge des einströmenden Fluids wird dabei beobachtet und dient als Hinweis auf die Funktionsfähigkeit der Fluidförderpumpe oder die Durchgängigkeit der Infusionsleitung bzw. des Hauptflussweges zwischen Pumpe und Messreservoir.

Unter einer Flussdrossel wird vorliegend allgemein ein Strömungswiderstand verstanden, um den Fluss zu reduzieren. Der Strömungswiderstand kann durch ein separates Bauteil oder durch einen geeigneten Querschnitt der Infusionsleitung geschaffen werden.

Nachdem der Durchfluss und die Funktionsfähigkeit der Infusionsleitungsanordnung anhand des Fluides in dem Messreservoir erkannt wurde, wird der Flussweg zum Patienten hin wieder geöffnet. Dabei strömt das in dem Messreservoir gesammelte Fluid aus dem Messreservoir heraus und wird durch den Hauptflussweg dem Patienten zugeführt. Es geht also durch die Messung des Durchflusses kein Fluid verloren.

Dabei kann das in dem separaten Messreservoir aufgenommene Fluid genutzt werden, um ein Prisma zu benetzen, um die Lichtbrechung des Prismas zu verändern. Beispielsweise kann das Prisma mit einer farbigen Schicht unterlegt sein, die nur dann sichtbar ist, wenn die Oberfläche des Prismas mit Fluid benetzt wird, während in trockener Umgebung der Strahlengang des Lichts innerhalb des Prismas dazu führt, dass die Farbschicht nicht sichtbar ist.

Ein anderes Prinzip zur Erkennung von Fluid innerhalb des Messreservoirs kann darin bestehen, dass in dem Messreservoir ein Kolben vorgesehen ist, der von einströmendem Fluid entgegen der Kraft einer Feder von außen sichtbar verschoben wird. Das von der Fluidförderpumpe in das Messreservoir eingepumpte Fluid verschiebt dann den Kolben. Die Verschiebung oder Auslenkung des Kolbens kann auf verschiedene Weise sichtbar gemacht werden. Beispielsweise kann der Kolben selbst sichtbar sein oder ein Anzeigeelement entlang einer Skala verschieben.

Eine weitere Alternative zur Erkennung von Fluid innerhalb des Messreservoirs kann in einer sichtbaren Formveränderung des Messreservoirs in Abhängigkeit von der Flüssigkeitsmenge in dem Reservoir bestehen. Beispielsweise kann es sich bei dem Messreservoir um einen Ballon handeln, der sich je nach Menge der Flüssigkeit ausdehnt. Ebenfalls denkbar sind ein Manometer, ein (Miniatur-) Faltenbalg oder ein elastischer Schlauch, der in leerem Zustand gekrümmt und in gefülltem Zustand gestreckt ist. Desweiteren soll für jede Art von Messreservoir denkbar sein, einen Zeiger vorzusehen, der über eine Hebelwirkung eine Verstärkung der Anzeige bewirkt.

Die erfindungsgemäße Vorrichtung zur Durchflusskontrolle kann insbesondere Teil einer PCA- (Patient Controlled Analgesia-) Vorrichtung zur patientenkontrollierten Analgese sein und/oder in Verbindung mit einem Flussselektor eingesetzt werden.

Bei der Fluidpumpe kann es sich um eine elastomere Pumpe, eine Federpumpe, eine Vakuumpumpe oder um eine Spritzenpumpe handeln.

Im Folgenden werden anhand der Figuren Ausführungsbeispiele näher erläutert. Es zeigen:
- Fig. 1: ein Ausführungsbeispiel welches nicht Teil der Erfindung ist in einem ersten Betriebszustand,
- Fig. 2: das Ausführungsbeispiel nach Fig. 1 in einem zweiten Betriebszustand,
- Fig. 3: ein Ausführungsbeispiel welches nicht Teil der Erfindung ist in einem ersten Betriebszustand,
- Fig. 4: das Ausführungsbeispiel nach Fig. 3 in einem zweiten Betriebszustand,
- Fig. 5: ein weiteres Ausführungsbeispiel welches nicht Teil der Erfindung ist in einem ersten Betriebszustand,
- Fig. 6: das Ausführungsbeispiel nach Fig. 5 in einem zweiten Betriebszustand,
- Fig. 7: ein Ausführungsbeispiel der Erfindung in einem ersten Betriebszustand,
- Fig. 8: das Ausführungsbeispiel nach Fig. 7 in einem zweiten Betriebszustand,
- Fig. 9: ein Detail eines weiteren Ausführungsbeispiels,
- Fig. 10: das Ausführungsbeispiel nach Fig. 9 in einem anderen Betriebszustand,
- Fig. 11: ein Detail eines weiteren Ausführungsbeispiels,
- Fig. 12: ein Detail eines weiteren Ausführungsbeispiels,
- Fig. 13: ein Detail eines weiteren Ausführungsbeispiels, welches nicht Teil der Erfindung ist,
- Fig. 14: ein Detail eines weiteren Ausführungsbeispiels, welches nicht Teil der Erfindung ist,
- Fig. 15: das Ausführungsbeispiel nach Fig. 14 in einem anderen Betriebszustand,
- Fig. 16: ein Detail eines weiteren Ausführungsbeispiels,
- Fig. 17: das Ausführungsbeispiel nach Fig. 16 in einem anderen Betriebszustand,
- Fig. 18: ein Detail eines weiteren Ausführungsbeispiels, welches nicht Teil der Erfindung ist, und
- Fig. 19: das Ausführungsbeispiel nach Fig. 18 in einem weiteren Betriebszustand.

Sämtliche Ausführungsbeispiele betreffen das Prinzip einer Infusionsleitungsanordung, die aus einer Infusionsleitung 12, einer Fluidförderpumpe 14 und einer Vorrichtung 16 zum Kontrollieren des Durchflusses durch die Infusionsleitung 12 besteht. Die Infusionsleitung 12 weist zwei Enden 18, 20 auf, von denen das erste Ende 18 mit der Fluidförderpumpe 14 verbunden ist und das zweite Ende 20 einem Patienten zugeordnet ist. Das zweite Ende 20 ist dadurch einem Patienten zugeordnet, dass es einen Konnektor 22 aufweist, der mit dem Patienten oder einer in dem Patienten eingeführten Infusionsleitung verbindbar ist.

Durch die Infusionsleitung 12 hindurch verläuft von deren einem Ende 18 hin zu deren anderem Ende 20 ein Hauptflussweg 24, entlang dem das Fluid von der Pumpe 14 zu dem Konnektor 22 hin gefördert wird.

Die Infusionsleitung 12 weist eine Abzweigung 26 auf, die mit einem Messreservoir 28 verbunden ist. Die Abzweigung 26 und das Messreservoir 28 bilden die Vorrichtung 16 zur Durchflusskontrolle. Hierbei kann die Abzweigung 26 als integraler Bestandteil der Infusionsleitung 12 oder als nachträglich mit der Infusionsleitung 12 verbindbare Einrichtung ausgebildet sein.

Die Abzweigung 26 bildet einen Messflussweg 30, der von dem Hauptflussweg 24 abzweigt und in das Messreservoir 28 mündet.

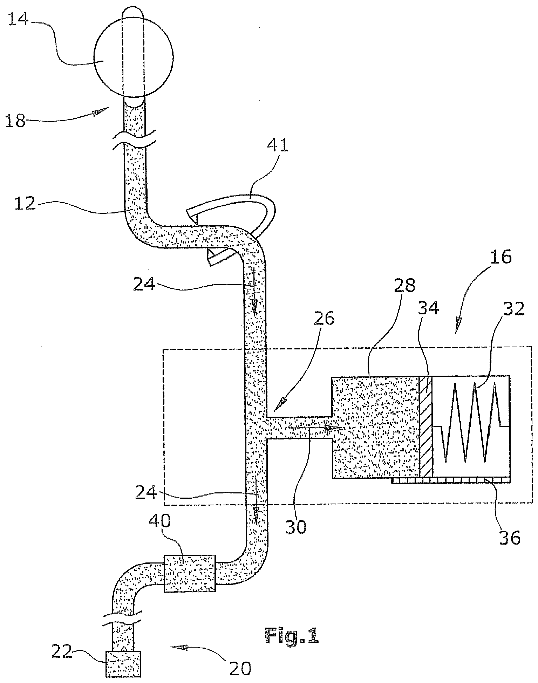

Bei dem Ausführungsbeispiel nach Fig. 1 ist in dem Messreservoir 28 ein entgegen der Kraft einer Feder 32 verschiebbarer Kolben 34 derart vorgesehen, dass das entlang des Messflussweges 30 strömende Fluid den Kolben 34 entgegen der Federkraft in dem Messreservoir 28 verschiebt. Die Auslenkung des Kolbens 34 wird auf einer Skala 36 angezeigt. Zwischen der Fluidpumpe 14 und der Abzweigung 26 ist die Infusionsleitung 12 mit einer Klemme 41 abklemmbar, um den Hauptflussweg 24 zu unterbrechen. Zwischen der Abzweigung 26 und dem Patientenkonnektor 22 weist die Infusionsleitung 12 eine Flussdrossel 40 auf.

In dem Betriebszustand nach Fig. 1 ist die Klemme 41 geöffnet und der Hauptflussweg 24 nicht unterbrochen. Die Fluidpumpe 14 fördert das gepunktet dargestellte Fluid entlang des Hauptflussweges 24 durch die Infusionsleitung 12 zu deren Patientenende 20 und über die Abzweigung 26 entlang des Messflussweges 30 in das Messreservoir 28. Dabei baut die Fluidpumpe 14 in dem Messreservoir 28 einen auf den Kolben 34 wirkenden Druck auf, der der Federkraft der Feder 32 entgegen wirkt und den Kolben 34 verschiebt. Die auf der Skala 36 sichtbare Auslenkung des Kolbens 34 kann als Indiz für den Betrieb oder die Funktionsfähigkeit der Fluidpumpe 14 dienen.

Um zu überprüfen, ob die Infusionsleitung 12, die Flussdrossel 40, der Patientenkonnektor 22 und mögliche weitere nachgeschaltete Komponenten wie z.B. Filter, Katheter usw. durchgängig und funktionsfähig sind, wird die Klemme 41 kurzzeitig geschlossen. Hierzu sollte die Klemme 41 keine Rastklemme sein, sondern bei Freigeben der Klemme 41 automatisch wieder öffnen. Alternativ kann die Infusionsleitung 12 auch kurzzeitig per Hand zusammen gedrückt oder geknickt werden, um den Fluss zu unterbrechen. Die Fluidpumpe 14 wird während des Abklemmens der Infusionsleitung 12 weiterbetrieben. Jedoch wird kein weiteres Fluid in das Messreservoir 28 gefördert und der von der Fluidpumpe 14 generierte Fluiddruck wirkt nicht mehr auf den Kolben 34. Die Federkraft verschiebt den Kolben und das Fluid wird aus dem Messreservoir 28 hinaus und in die Infusionsleitung 12 in Richtung des Patientenendes 20 hin gefördert, wenn die Infusionsleitung und sämtliche nachfolgenden Komponenten durchgängig sind. Mit durchgängig ist dabei gemeint, dass das Fluid gefördert wird und der Fluidstrom nicht durch Beschädigungen, Knicke oder Verstopfungen blockiert oder vermindert wird. Die Auslenkung des Kolbens 34 dient dabei als Maß für die Durchgängigkeit der Infusionsleitung 12 entlang des Hauptflussweges 24 in Richtung des Patienten. Falls die Infusionsleitung 12 oder eine der mit ihr verbundenen Komponenten beschädigt ist und den Fluidstrom sperrt, wird der Kolben 34 weniger oder kein Fluid aus dem Messreservoir 28 herauspressen. Die Auslenkung des Kolbens 34 ist dann eine andere als im Falle einer durchgängigen Infusionsleitung 12.

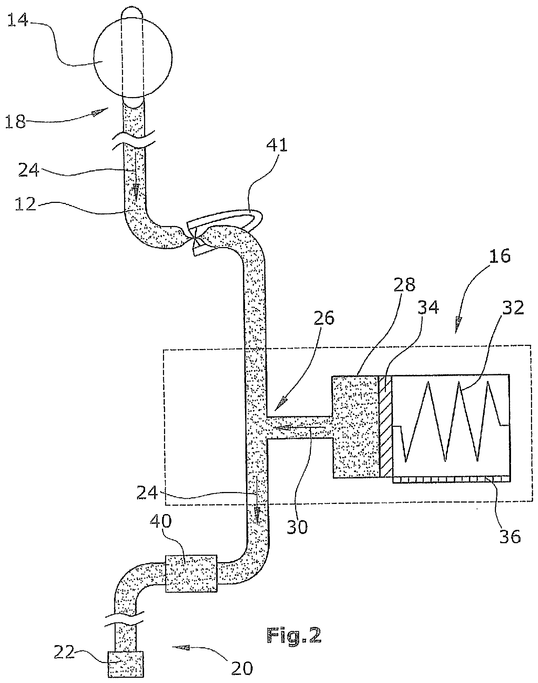

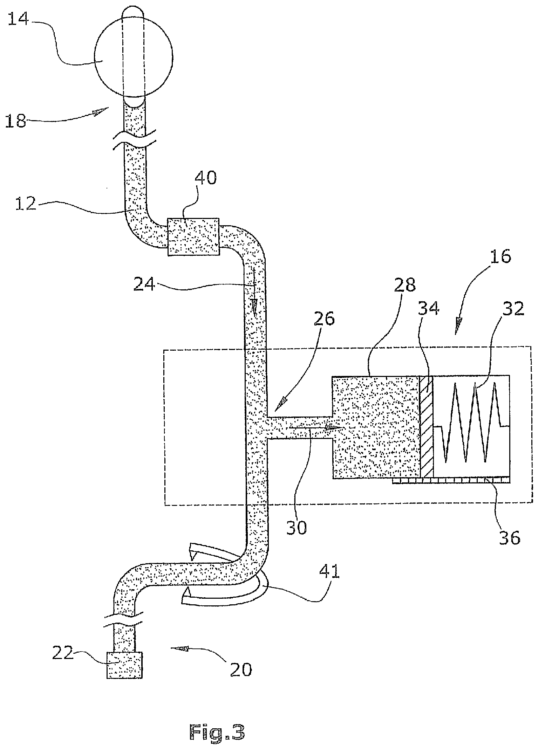

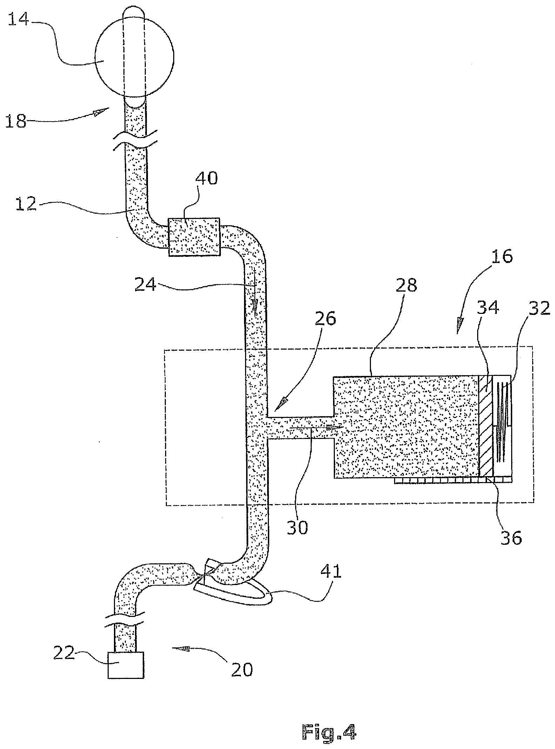

Das Ausführungsbeispiel nach den Fign. 3 und 4 unterscheidet sich von dem Ausführungsbeispiel nach den Fign. 1 und 2 lediglich in der Anordnung der Klemme 41 und der Flussdrossel 40. Bei dem zweiten Ausführungsbeispiel ist die Flussdrossel 40 zwischen der Fluidpumpe 14 und der Abzweigung 26 angeordnet. Die Klemme 41 dient zur Unterbrechung des Hauptflussweges 24 im Bereich zwischen der Abzweigung 26 und dem Patientenende 20. Im nicht abgeklemmten Zustand gemäß Fig. 3 entspricht der Betriebszustand dann demjenigen nach Fig. 1. Die Klemme dient und kann zum einen als Kontrolleinheit und zum anderen als Vortäuschung einer Blockade gesehen werden.

Der zweite Betriebszustand gemäß Fig. 4 unterscheidet sich jedoch von dem zweiten Betriebszustand des ersten Ausführungsbeispiels gemäß Fig. 2. In Fig. 4 wird bei abgeklemmter Infusionsleitung 12 die Fluidpumpe 14 weiter betrieben und Fluid auch weiterhin in das Messreservoir 28 gefördert. Dadurch, dass kein Fluid mehr in Richtung des Patientenendes 20 strömen kann, baut die Fluidpumpe 14 einen immer größer werdenden Fluiddruck innerhalb des Messreservoirs 28 auf. Die dadurch erfolgende Auslenkung des Kolbens 34 dient dann als Hinweis auf die Funktionsfähigkeit der Fluidpumpe 14 und die Durchgängigkeit der Infusionsleitung 12 im Bereich zwischen der Fluidpumpe 14 und dem Messreservoir 28. Die Klemme 41 (als Kontrolleinheit) wird auch in Fig. 4 nur kurzzeitig geschlossen, damit die Unterbrechung der Infusion kurz ist und die Gesamtmenge des dem Patienten verabreichten Fluides nicht abnimmt. Bei einer Blockade (am Patienten) funktioniert die Einheit "automatisch" (Flüssigkeitssäule würde steigen).

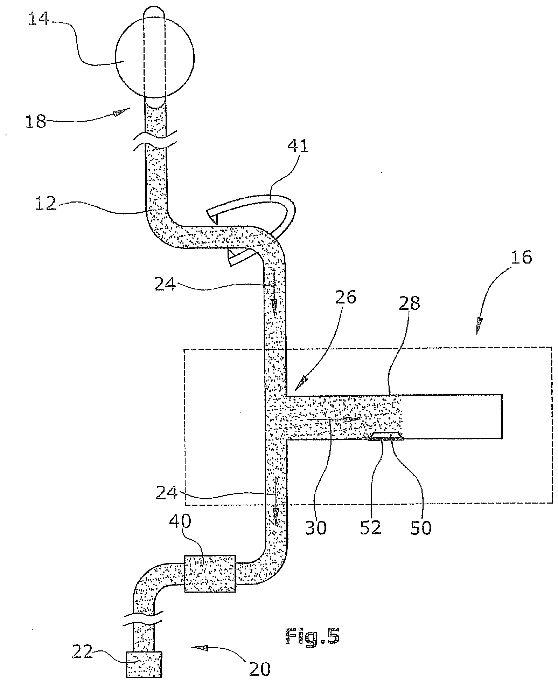

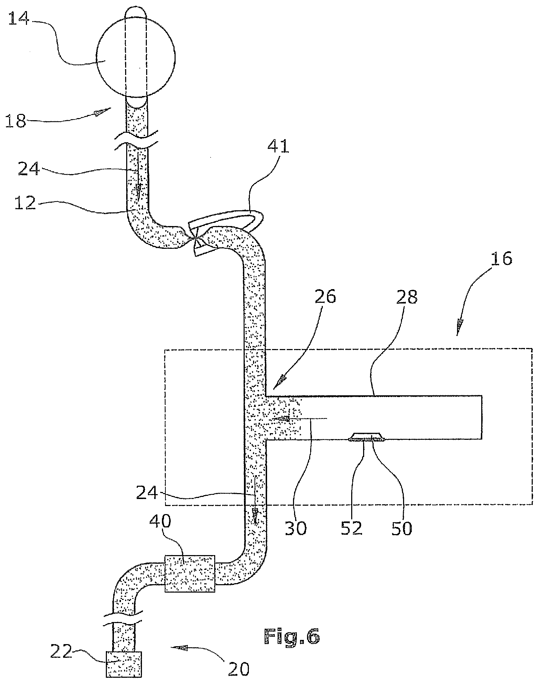

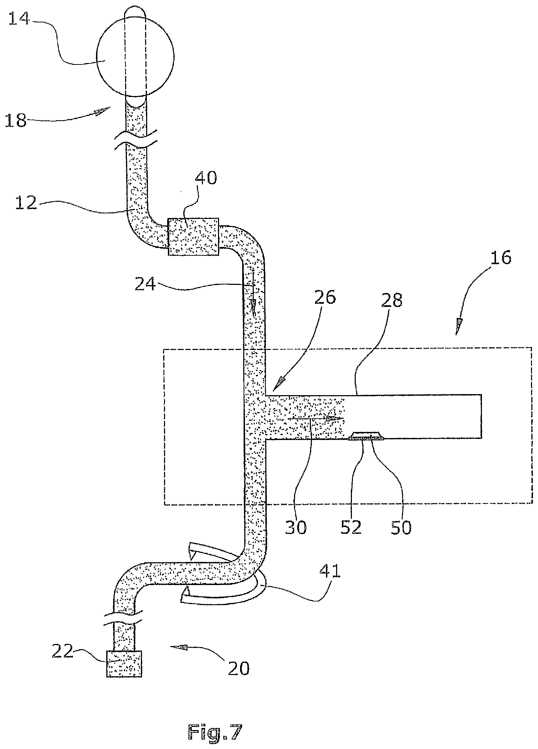

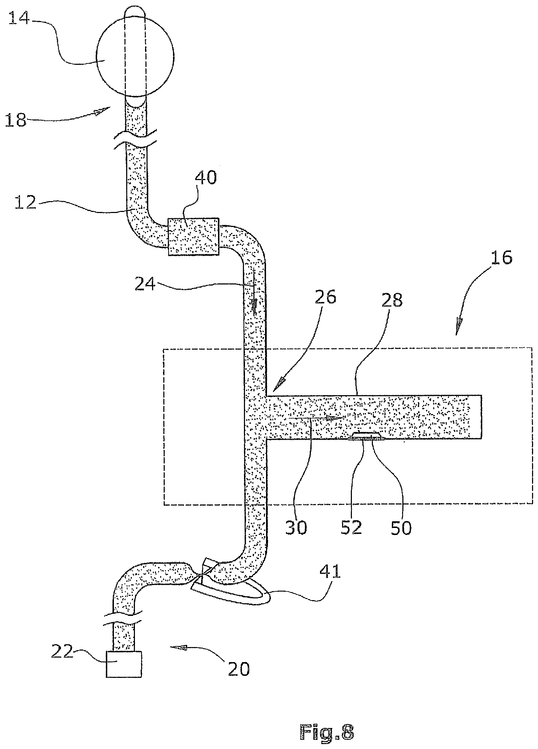

Das dritte Ausführungsbeispiel gemäß den Fign. 5 und 6 entspricht bis auf die Vorrichtung 16 zur Kontrolle des Durchflusses dem ersten Ausführungsbeispiel gemäß den Fign. 1 und 2. Entsprechend unterscheidet sich das vierte Ausführungsbeispiel gemäß den Fign. 7 und 8 von dem zweiten Ausführungsbeispiel gemäß den Fign. 3 und 4 lediglich durch die Vorrichtung 16. Die Vorrichtungen 16 zur Kontrolle des Durchflusses sind in den Ausführungsbeispielen 3 und 4 gleich. Der Unterschied besteht gegenüber den Ausführungsbeispielen 1 und 2 darin, dass das Messreservoir keinen entgegen der Kraft einer Feder 32 verschiebbaren Kolben 34 aufweist, sondern vielmehr ein Prisma 50, an dessen Unterseite eine Farbschicht 52 beispielsweise in roter Farbe ausgebildet ist. Das Prisma ist lichtdurchlässig und derart ausgebildet, das es in dem in Fig. 6 dargestellten Zustand in trockener Umgebung Licht total reflektiert, so dass die Farbschicht 52 nicht sichtbar ist. In den in den Fign. 5 und 8 dargestellten Zuständen ist das Prisma 50 von dem Fluid benetzt, wobei keine Totalreflektion mehr erfolgt und die Farbschicht 52 sichtbar ist. Dies hat zur Folge, dass bei funktionierendem, ausreichendem Durchfluss die Vorrichtung 16 aufgrund der speziellen Brechungsverhältnisse des Prismas 50 die Farbschicht 52 als Indikator für korrekten Durchfluss anzeigt. Wenn das Messreservoir 28, wie beispielsweise in Fig. 6 oder im Falle einer defekten Fluidförderpumpe kein Fluid enthält und das Prisma 50 in trockener Umgebung enthalten ist, wird die Farbschicht 52 hingegen nicht angezeigt.

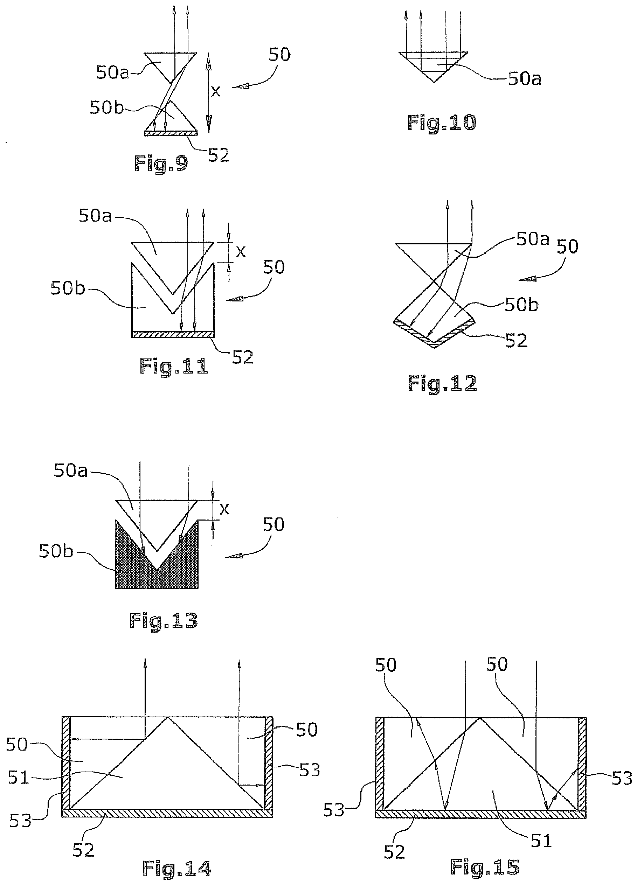

Die Fign. 9 - 15 zeigen verschiedene Ausführungsbeispiele eines solchen Prismas 50. Die Fign. 9 - 13 zeigen dabei zweiteilige Prismen 50 mit einem Oberteil 50a und einem Unterteil 50b. Die Unterseite des Prismenunterteils 50b ist in den Fign. 9, 11 und 12 mit einer Farbschicht 52 versehen. In Fig. 13 ist eine separate Farbschicht nicht vorhanden, sondern das Prismenunterteil 50b ist vielmehr farbig ausgebildet. Die Fign. 9, 11, 12 und 13 zeigen den Strahlengang des Lichts durch das Prisma 50 bei Benetzung des Prismas mit Fluid, das heißt also in den in den Fign. 5 und 8 dargestellten Betriebszuständen. Fig. 10 zeigt das Prismenoberteil 50a gemäß dem Ausführungsbeispiel nach Fig. 9 in trockener Umgebung, in der das Licht total reflektiert wird und die Farbschicht 52 nicht sichtbar ist. Dies ist in dem Betriebszustand nach Fig. 6 der Fall.

Die Fign. 14 und 15 zeigen ein Ausführungsbeispiel eines zweiteiligen Prismas 50, dessen zwei Teile zusammen mit der farbigen Unterseite 52 einen Durchströmkanal 51 für das Fluid umschließen. Das Prisma ist in dem Messreservoir 28 dabei derart angeordnet, dass in dem Messreservoir 28 enthaltenes Fluid in den Kanal 51 einströmt. Fig. 14 zeigt den Strahlengang in dem Betriebszustand nach den Fign. 6 und 7, das heißt in trockener Umgebung. Dabei reflektiert das Prisma 50 das einfallende Licht auf eine seitliche Farbschicht 53 in zum Beispiel roter Farbe. In trockener Umgebung gemäß Fig. 6 ist also die rote Farbe sichtbar. Fig. 15 zeigt den Strahlengang in den Betriebszuständen nach den Fign. 5 und 8, in denen der Kanal 51 fluiddurchströmt ist. Dies führt zu dem in Fig. 15 dargestellten Strahlengang, bei dem das Licht an der farbigen Unterseite 52 reflektiert wird. Die Farbschicht 52 ist dabei in zum Beispiel grüner Farbe gehalten, so dass das Prisma in Fig. 15 die grüne Farbe anzeigt.

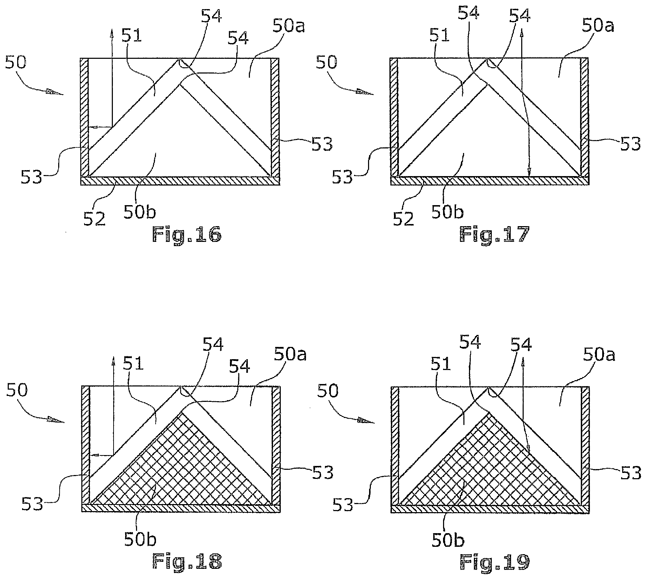

Die Fign. 16 und 17 zeigen ein Ausführungsbeispiel eines zweiteiligen Prismas 50, das aus zwei Teilprismen 50a und 50b besteht. Alle Prismen 50a, 50b sind jeweils rechtwinklig, das heißt mit einer rechtwinkligen Spitze 54 versehen. Zwischen den beiden Prismen 50a, 50b ist ein Kanal 51 zum Durchströmen des Fluids vorgesehen. An den seitlichen Rändern ist eine Farbschicht 53 einer ersten Farbe (zum Beispiel rot) vorgesehen, die das Licht in dem in Fig. 16 dargestellten trockenen Zustand reflektiert. In trockener Umgebung ist somit die rote Farbschicht zu sehen. Fig. 17 zeigt den Strahlengang, wenn Fluid in dem Kanal 51 enthalten ist. Das einfallende Licht wird dann auf eine untere Farbschicht 52 einer zweiten, von der ersten Farbe verschiedenen Farbe (grün) reflektiert. Wenn Fluid in dem Kanal 51 enthalten ist, ist somit die grüne Farbschicht sichtbar.

Das Ausführungsbeispiel nach den Fign. 18 und 19 unterscheidet sich von dem Ausführungsbeispiel nach den Fign. 16 und 17 lediglich dadurch, dass keine untere Farbschicht 52 unter dem zweiten Teilprisma 50b vorgesehen ist, sondern stattdessen das zweite Prisma 50b vielmehr in der zweiten, von der ersten verschiedenen Farbe (grün) eingefärbt ist. In dem in Fig. 18 dargestellten trockenen Zustand ohne Fluid in dem Kanal 51 wird, wie in Fig. 16, das Licht von der roten Farbschicht 53 reflektiert. Bei dem in Fig. 19 dargestellten Zustand mit durchströmten Kanal 51 wird das Licht von dem grünen Teilprisma 50 reflektiert und die grüne Färbung des Prismas 50b ist sichtbar.

## Patentansprüche

1. Infusionsleitungsanordnung mit einer Infusionsleitung (12), an deren einem Ende (18) eine Fluidförderpumpe (14) vorgesehen ist und deren anderes Ende (20) einem Patienten zugeordnet ist, wobei die Infusionsleitung (12) einen Hauptflussweg (24) von der Fluidförderpumpe (14) zu dem Patientenende (20) bildet, und mit einer Durchflusskontrollvorrichtung, die ein Messreservoir (28) und eine einen Messflussweg (30) bildende Abzweigung (26) aufweist, die von dem Hauptflussweg (24) abzweigt und in das Messreservoir (28) mündet, wobei die Infusionsleitung (12) zwischen der Fluidförderpumpe (14) und der Abzweigung (26) eine Flussdrossel aufweist, und wobei in dem Messreservoir (28) ein von in das Messreservoir (28) einströmendem Fluid benetzbares Prisma (50) vorgesehen ist, das Licht bei Fluidbenetzung anders bricht als in trockener Umgebung,
**dadurch gekennzeichnet, dass**
das Prisma (50) zweiteilig gestaltet ist und ein Prismenoberteil (50a) sowie ein mit einer Farbschicht (52) versehenes Prismenunterteil (50b) aufweist.

## Claims

1. Infusion line arrangement, comprising an infusion line (12) with a fluid supply pump (14) provided at one end (18) thereof and the other end (20) thereof related to a patient, wherein the infusion line (12) forms a main flow path (24) from the fluid supply pump (14) to the patient end (20), and comprising a flow control device having a metering reservoir (28) and a branching (26) forming a metering flow path (30), the branching branching-off from the main flow path (24) and opening into the metering reservoir (28), wherein the infusion line (12) has a flow restrictor between the fluid supply pump (14) and the branching (26), and wherein within the metering reservoir (28) a prism (50) is provided which can be wetted by fluid flowing into the metering reservoir (28), and which prism exhibits a different refraction of light when wetted with fluid than in a dry environment,
**characterized in that**
the prism (50) has a two-part design and includes a prism top part (50a) and a prism bottom part (50b) provided with a colour layer (52).

## Revendications

1. Agencement de conduit d'infusion comprenant un conduit d'infusion (12) au niveau d'une extrémité (18) duquel est prévue une pompe de refoulement de fluide (14) et dont l'autre extrémité (20) est affectée à un patient, le conduit d'infusion (12) formant une voie d'écoulement principale (24) allant de la pompe de refoulement de fluide (14) à l'extrémité patient (20), et comprenant un dispositif de contrôle de débit qui présente un réservoir de mesure (28) et une ramification (26) formant une voie d'écoulement de mesure (30) qui part de la voie d'écoulement principale (24) et débouche dans le réservoir de mesure (28), le conduit d'infusion (12) présentant un étranglement d'écoulement entre la pompe de refoulement de fluide (14) et la ramification (26), et un prisme (50) pouvant être mouillé par le fluide affluant dans le réservoir de mesure (28) étant prévu dans le réservoir de mesure (28), lequel réfracte la lumière lorsqu'il est mouillé par le fluide de manière différente que dans un environnement sec, **caractérisé en ce que**
le prisme (50) est configuré en deux parties et présente une partie supérieure de prisme (50a) ainsi qu'une partie inférieure de prisme (50b) pourvue d'une couche colorée (52).
